(19) Europäisches Patentamt
European Patent Office
Office européen des brevets

(11) Publication number: **0 377 958 A2**

(12) # EUROPEAN PATENT APPLICATION

(21) Application number: 89312272.1

(51) Int. Cl.[5]: C07D 501/02

(22) Date of filing: 27.11.89

(30) Priority: 10.01.89 ES 8900071

(43) Date of publication of application:
18.07.90 Bulletin 90/29

(84) Designated Contracting States:
AT BE CH DE ES FR GB GR IT LI LU NL SE

(71) Applicant: ANTIBIOTICOS, S.A.
Antonio Lopez 111
E-28026 Madrid(ES)

(72) Inventor: Nieves Elvira, Rosa Maria
Lino 11
E-28020 Madrid(ES)
Inventor: Conde Ruzafa, Santiago
Antonio Perez 13
E-28002 Madrid(ES)
Inventor: Fernandez Lizarbe, Jose Ramon
Artesa de Segre 12
E-28035 Madrid(ES)

(74) Representative: Ablewhite, Alan James et al
MARKS & CLERK 57/60 Lincoln's Inn Fields
London WC2A 3LS(GB)

(54) **Process for the preparation of 3-chloro-cefem compounds.**

(57) The process comprises a series of stages essentially comprising the protection of the functional groups of the side chain of Ampicillin, esterification of the acid group, oxidation to sulphoxide, expansion of the thiazole ring to a thiazine ring, ozonolysis of the exomethylene cefam derivative obtained, substitution of the hydroxyl group by chlorine and de-protection of the functional groups, in which the order of some stages can be varied without affecting the final result.

EP 0 377 958 A2

# Process for the preparation of 3-chloro-cefem compounds

## Technical Field of the Invention

The present invention relates to a process for preparing a 3-chloro-cefem compound, its esters and pharmaceutically acceptable salts, starting from a compound which can be easily dried as it is 6-(α-amino-phenylacetamide)-penicillanic acid, by means of a sequence of reactions on the amino-phenylacetamide group, which leads to a 3-chloro-cefem with the amino-phenylacetamide grouping in position 7, thereby producing compounds of importance in human and veterinary medicine.

## State of the Art

The procedures hitherto described for the preparation of 3-chloro-cefem compounds, to which the present invention relates, start from penam or cefem compounds, in which the side chain is inter alia phenoxyacetyl or thienylacetyl, which has to be removed in order to be substituted by α-aminophenylacetyl, by means of hydrolysis and subsequent acylation.

The present invention introduces the novelty that the entire sequence of reactions which leads to the aforesaid 3-chloro-cefem compounds is performed with the radical α-amino-phenylacetyl duly protected, which appreciably reduces the number of stages required for its preparation, simplifying its industrial application.

## Detailed Description of the Invention

The present invention relates to a process for the preparation of 3-chloro-cefem compounds and, more particularly, (6R,7R)-7-[(R)-2-amino-2-phenylacetamido]-3-chloro-8-oxo-5-thia-1-azabicyclo[4,2,0]-oct-2-ene-2-carboxylic acid, as well as its salts or derivatives of therapeutic or industrial application, characterized by the general formula

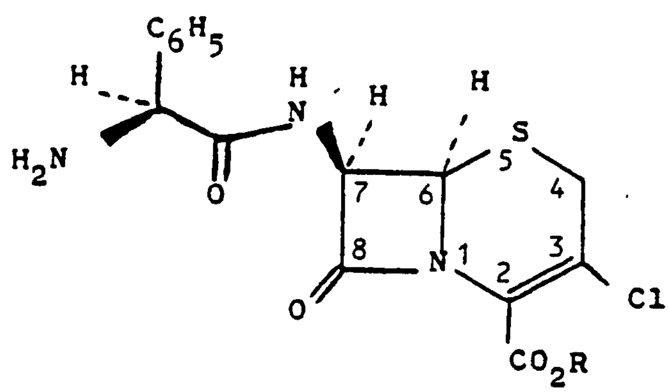

(I)

in which R represents a hydrogen atom or a therapeutically acceptable organic or metallic cation.

The compound of the general formula has been named in the first paragraph in accordance with the official rules of nomenclature of IUPAC (International Union of Pure and Applied Chemistry) based on the number system:

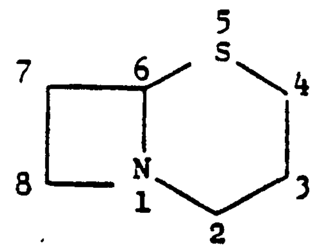

of the heterocyclic system 5-thia-1-azabicyclo[4,2,0]octane, corresponding to derivatives of cephalosporin, in the same way that the system 4-thia-1-azabicyclo[3,2,0]heptane and the number system:

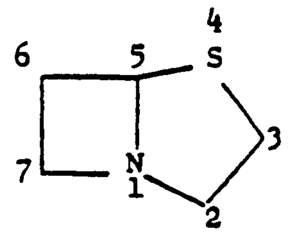

corresponds to derivatives of penicillin. From here onwards the following number systems will be used:

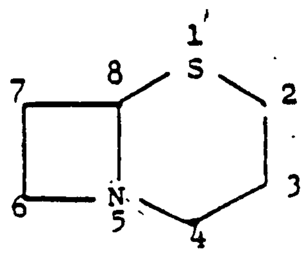

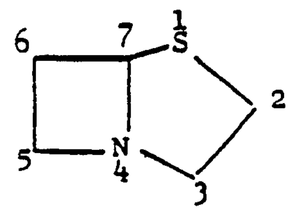

derived from the cefam and penam systems, much more common in the field of antibiotics, and the official nomenclature of IUPAC is only used in the titles of the compounds in the experimental part.

The compounds of formula I, of which a preparation process is claimed, include the known antibiotic Cefaclor in which R = H, described in US 3 925 372. It is a cephalosporin of wide antibacterial range as described in Antimicrob. Agents Chemother. 12 591 (1977) and 14 614 (1978). This antibiotic has the important characteristic that, in addition to being active in parenteral administration, for example subcutaneous or intramuscular, it is also effective when administered orally. This property makes it particularly useful in the treatment of bacterial infections, both in human and equally in veterinary medicine.

The procedures described up till now are based on obtaining compounds of the general formula II

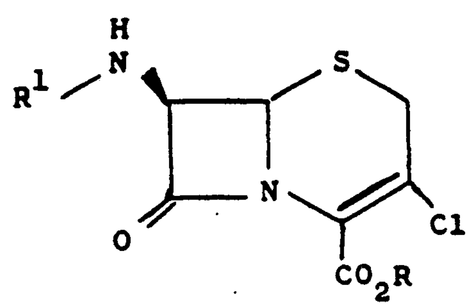

(II)

in which R has the value indicated above and $R^1$ represents an acylating group, in general one of those generally used in the field of penicillins and cephalosporins, for example phenoxyacetyl or thienylacetyl, of formulae III and IV respectively.

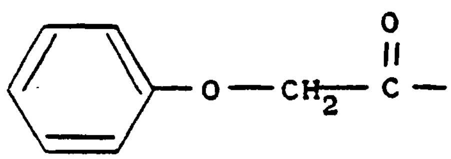

(III)

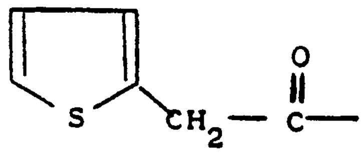

(IV)

These compounds of general formula II must undergo a process of hydrolysis to eliminate the acylating chain of the amine group in position 7, normally the radicals III or IV, to produce the basic compounds of general formula V:

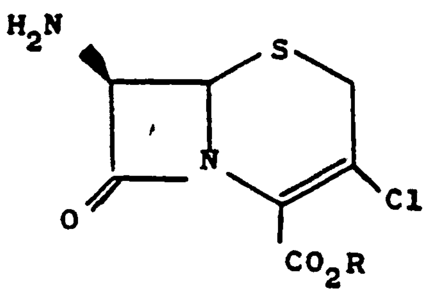

(V)

These compounds V must be subjected to a new acylation to introduce the radical D-2-amino-2-phenylacetyl VI:

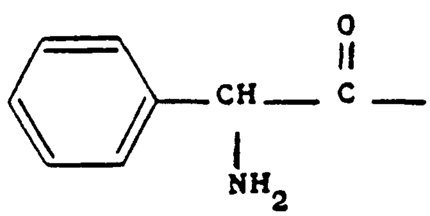

(VI)

and finally yield the already mentioned compounds of the general formula I.

The procedure described in the present invention provides the interesting novelty that the entire sequence of reactions which leads to the compounds of the general formula I is carried out with the radical D-2-amino-2-phenylacetyl (VI), duly protected, as an acylating chain of the amine group at 7 ($R^1$ in II). In this way, the long sequence of reactions is substantially shortened, by avoiding the hydrolysis of the side chain of II and the subsequent acylation of V.

The chemical name of the starting product for this synthesis according to the systematic nomenclature of IUPAC is 6-[(R)-2-amino-2-phenylacetamido]-3,3-dimethyl-7-oxo-4-thia-1-azabicyclo[3,2,0]heptane-2- carboxylic acid (VII) or 6-($\alpha$-aminophenyl-acetamido)- penicillanic acid, a very well-known and easily available semi-synthetic penicillin.

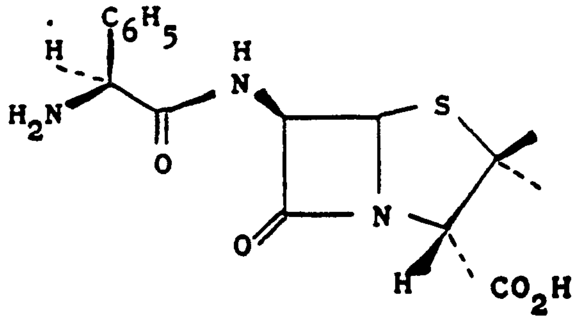

(VII)

The procedure which forms the subject of this patent is characterized by the following stages:

Stage 1

Ampicillin acid is dissolved in acetone with a basic compound, preferably a tertiary amine, to produce an imidazole derivative of formula VIII:

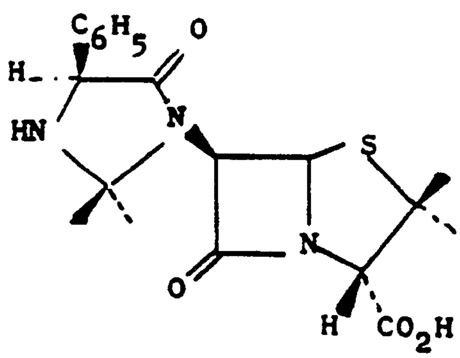

(VIII)

This compound VIII is treated, without previous purification with sodium nitrite, in a two-phase system, at a moderately acid pH, to provide high yields of a crystalline product, the formula of which is represented as IX:

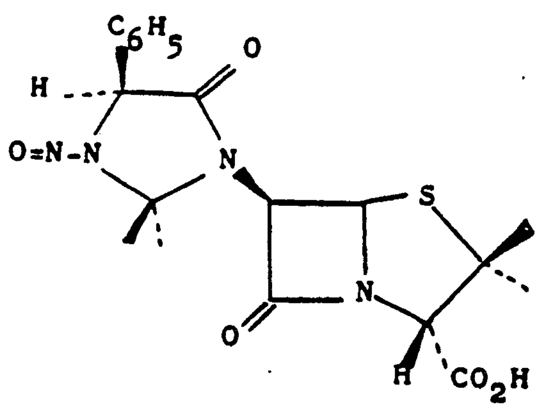

(IX)

Stage 2

The compound IX is esterified by reaction with a halogen derivative, such as an alkyl or benzyl halide, in an aprotic polar medium, such as that provided by the solvents dioxan, tetrahydrofuran, acetone, N,N-dimethylformamide or mixtures thereof. This solution is treated with a weak alkaline agent, such as sodium bicarbonate or potassium bicarbonate, compounds of general formula being obtained:

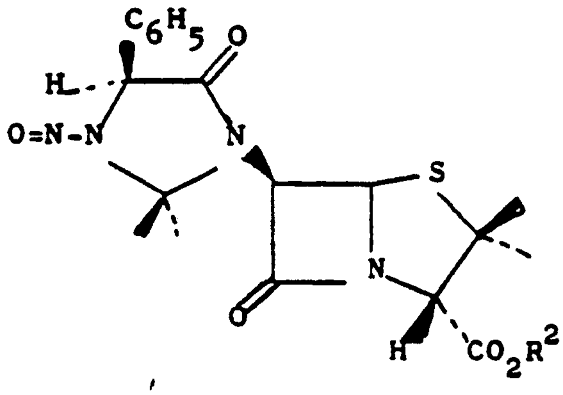

(X)

The radical represented by $R^2$ forms an ester group with the carboxylic acid in position 3 of the penam bicyclic system and should have the characteristics of giving greater stability to the molecule, blocking or protecting the carboxyl group and enhancing the solubility of the various compounds which are formed in the sequence of reactions constituting the subject of the present invention. In addition, it should be easy to eliminate by a simple chemical reaction such as reduction, or chemical or enzyme hydrolysis. By way of example, the chemical groups methyl, benzyl or para-nitrobenzyl can be named. These examples should not be taken as a limitation of the patent, since any other group which meets the requirements set out above is also included in the patented procedure.

This radical $R^2$ retains the value indicated in the preceding paragraph in the description of the following stages of the procedure forming the subject of the patent.

These compounds need not be separated and purified, but are directly subjected to oxidation by treatment of the solution of the said esters X with a per-acid or peroxide, in any of the solvents normally used in these reactions, such as methylene chloride, methanol, acetone, or mixtures thereof, cooled to low temperature. High yields of the (R) sulphoxides of the general formula XI are obtained:

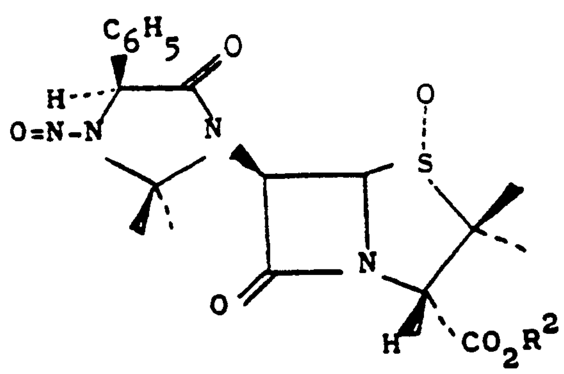

(XI)

These sulphoxides are separated as crystalline solids having precise melting points.

## Stage 3

This is characterized by the expansion of the 5-membered penam ring to the six membered cefem, by way of a monocyclic intermediary which it is not necessary to separate.

The sulphoxides of general formula XI, obtained in the previous stage, are dissolved in a hydrocarbon solvent such as benzene or toluene, or a chlorinated solvent such as chloroform or 1,2-dichloroethane, and are made to react by heating with an N-haloamide or N-haloimide, such as N-chloroacetamide, N

chlorosuccimide or N-chlorophthalimide. This treatment opens the thiazolidine ring, producing a monocyclic sulphinyl chloride of the general formula XII which is not separated:

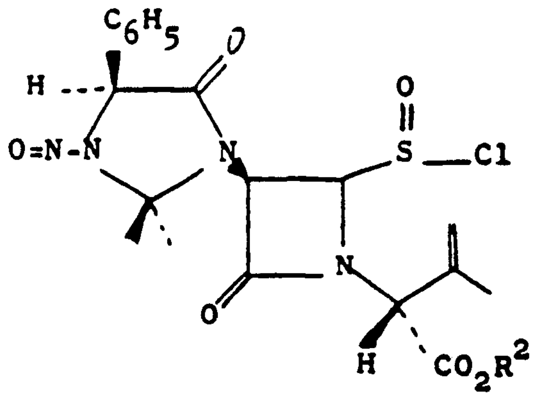

(XII)

and which is immediately treated with a compound of the group known in chemistry as Lewis acids, such as aluminium chloride, zinc bromide or tin (IV) chloride. The reaction product is precipitated as an aggregation complex with a metallic salt. Starting from this complex, of unknown formula, the 3-exomethylene cefam-S-oxide of the general formula XIII is obtained,

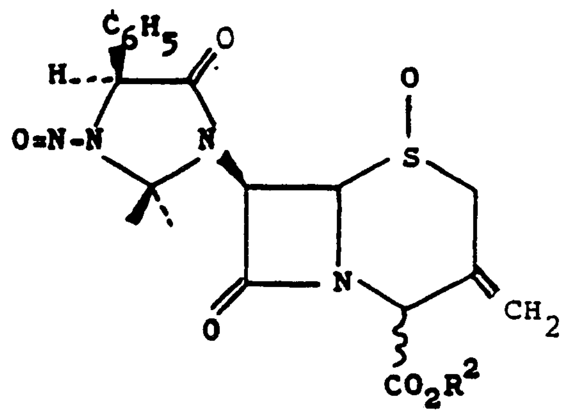

(XIII)

in which $R^2$ has the same value as a mixture of both isomeric sulphoxides R- and S-. The separation of the sulphoxides, starting from the metallic complex, is carried out by treatment of the latter with any molecule with free electron pairs, such as alcohols (methanol, ethanol, isopropanol) or ethers (diethyl ether. tetrahydrofuran).

## Stage 4

The mixture of the two S-oxides or isomeric sulphoxides of the 3-exomethylene cefam XIII is subjectec to ozonolysis, in solution in any solvent customary for these reactions, such as methanol, acetone methylene chloride or mixtures thereof. This reaction is carried out at low temperature, in a bath o acetone/dry ice. The ozonide formed is destroyed by any of the usual chemical procedures, such as fo example treatment with sulphur dioxide, sodium bisulphite, dimethyl sulphide, possibly including simple heating. High yields of the mixture of isomeric S-oxides of the 3-hydroxycefem compound of formula XIV are obtained:

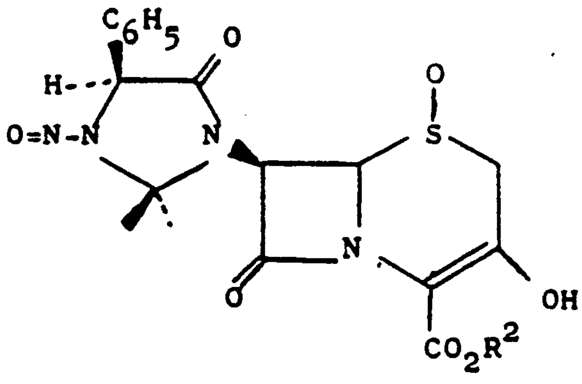

(XIV)

Stage 5

This stage is characterized by the fact that three reactions take place which are effected by the use o the Vilsmeier reagent, which acts simultaneously as a halogenating agent and as a reducing agent. B) treating the 3-hydroxy cefem-5-oxide XIV with the aforesaid reagent, the hydroxyl group in position 3 of the cefem ring undergoes a 3-chloro substitution reaction, resulting in the product of the general formula XV

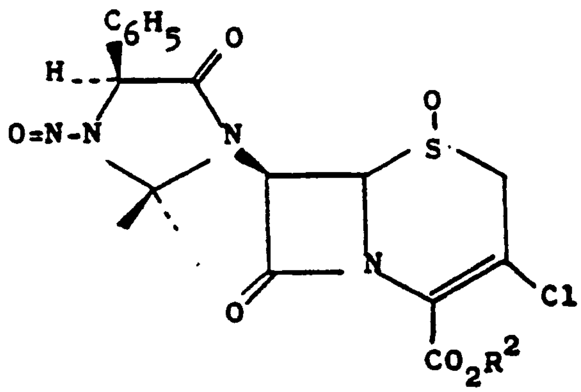

(XV)

which can be separated when work is performed at low temperatures. Under normal working conditions tw other reactions take place simultaneously: the reduction of the S-oxide group to sulphide and the reductive denitrosation of the nitroso group which protects the secondary amine which constitutes position 3 of the imidazolidine ring in the side chain, a compound of formula XVI being produced in a single stage:

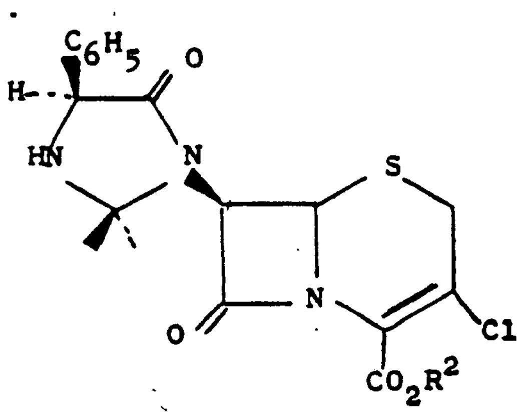

(XVI)

This product is in fact the molecule of the required antibiotic with protective or blocking groups on the carboxyl and on the side chain.

Stage 6

In this final stage the compound XVI is subjected to deblocking of the carboxyl group, by any type of reduction, or hydrolysis, according to the nature of the esterifying radical $R^2$. In the case of hydrolysis deprotection of the side chain also takes place simultaneously to produce the required antibiotic in the form of a monohydrate, by precipitation at its iso-electric point. In the case where deblocking of the carboxyl group is carried out by hydrogenolysis, the subsequent hydrolysis is performed in a single step, without separating or purifying any intermediate product. In this final stage the compound I is obtained in which R represents hydrogen which can subsequently be substituted by an alkali metal or any other salt, inorganic or organic.

The overall chemical reaction scheme which constitutes the process forming the subject of the present invention, separated into its various stages, is as follows:

Stage 1

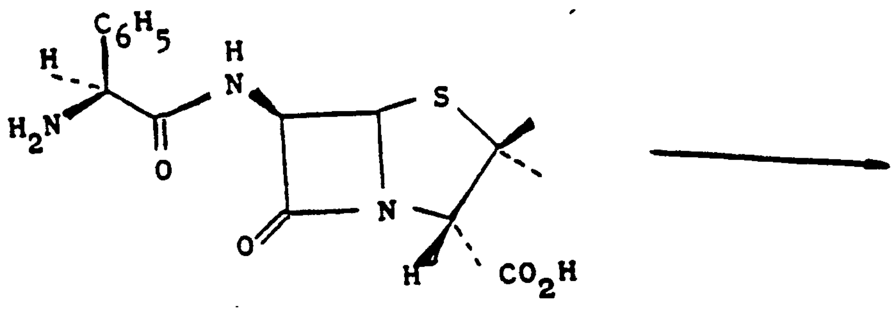

VII

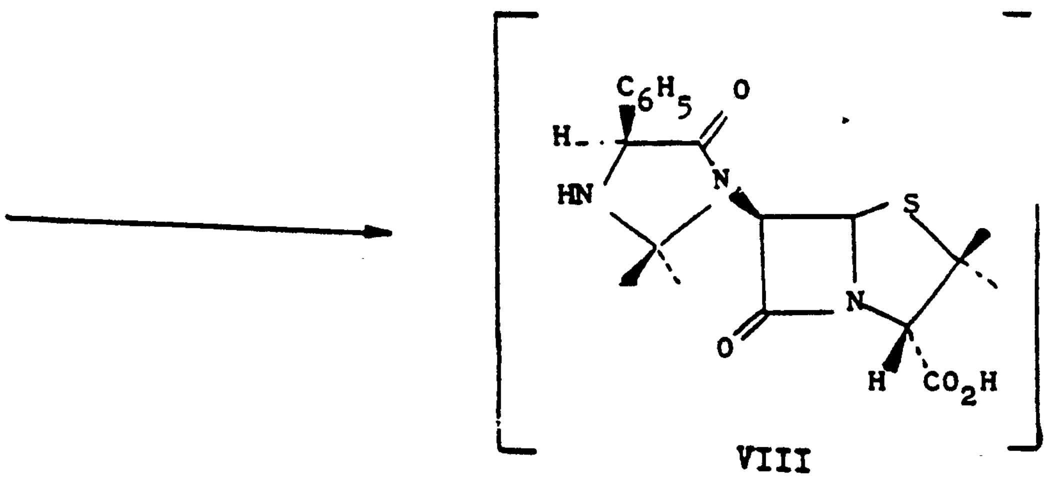

VIII

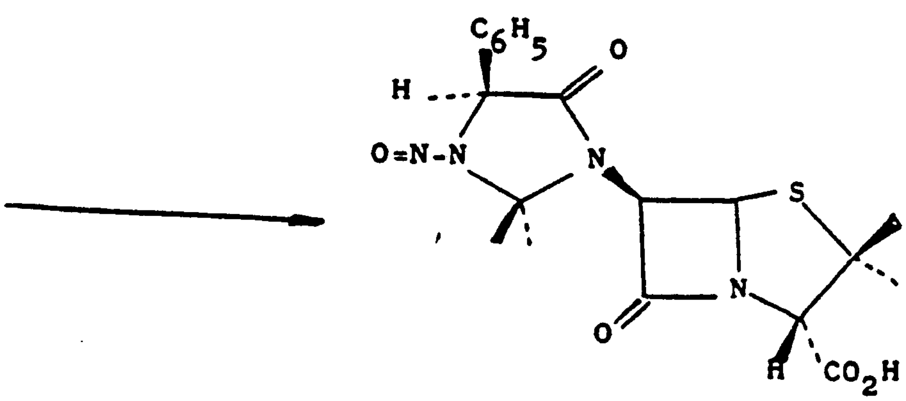

IX

Stage 2

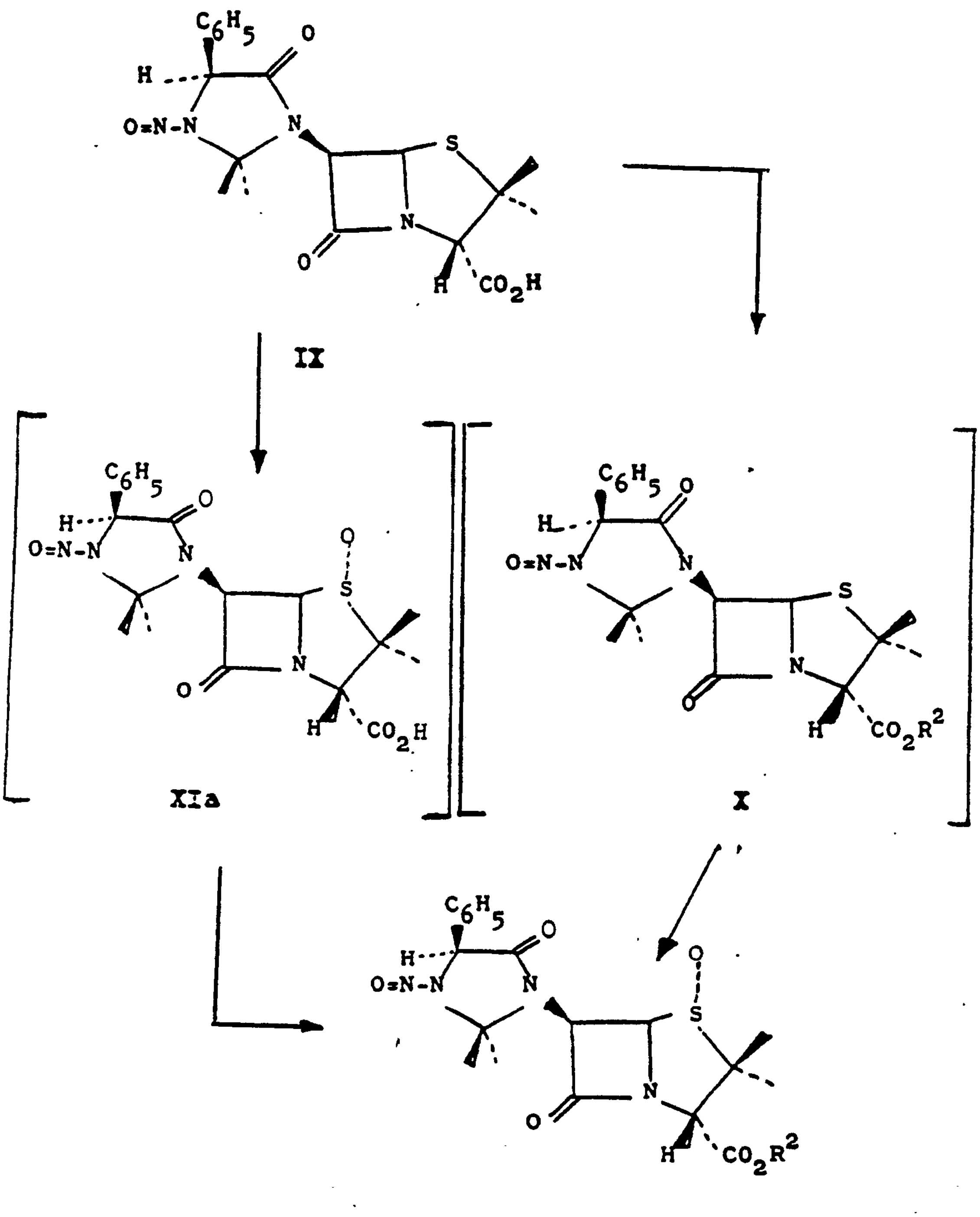

IX

XIa

X

XI

Stage 3

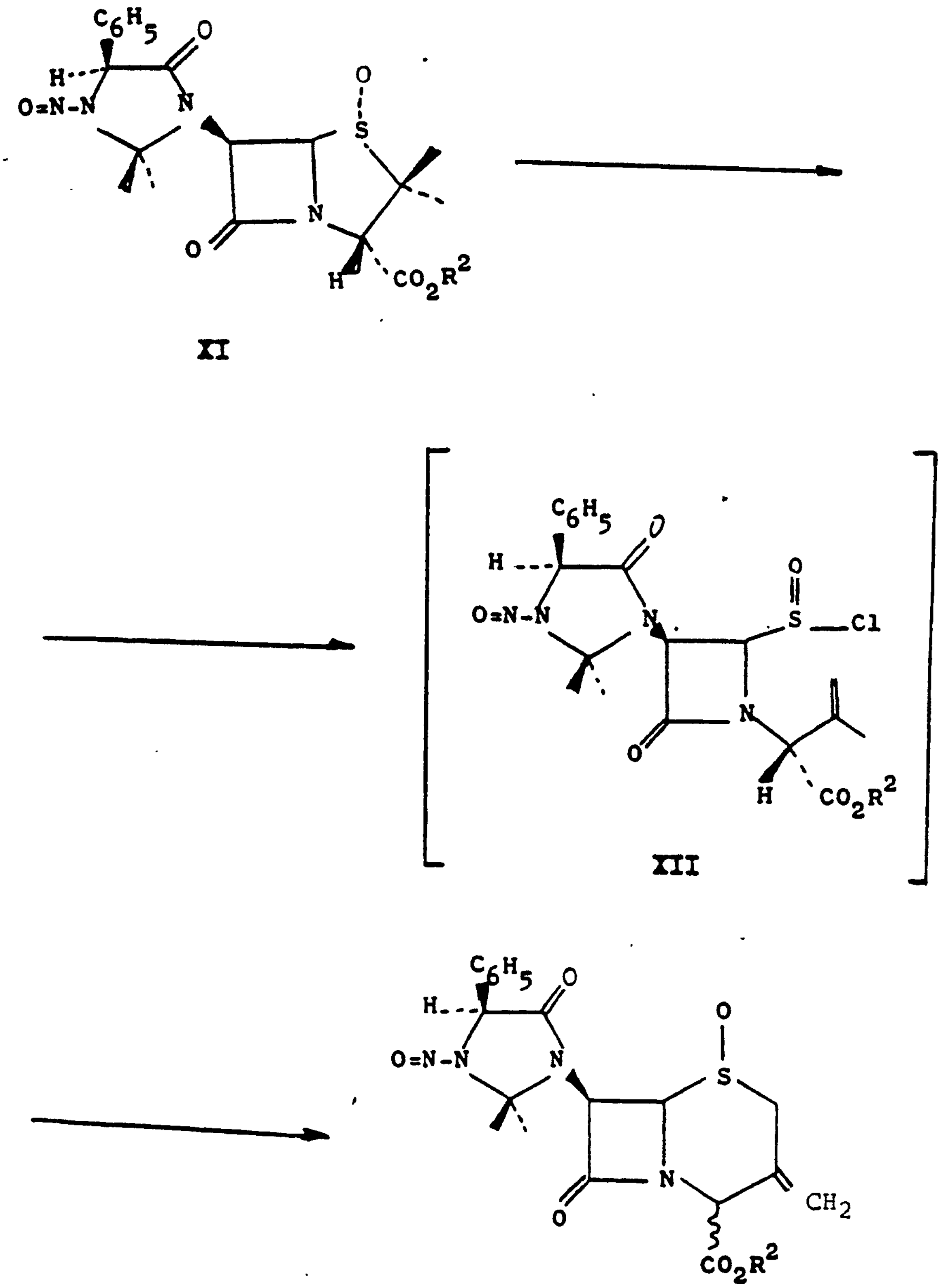

XI

XII

XIII

Stage 4

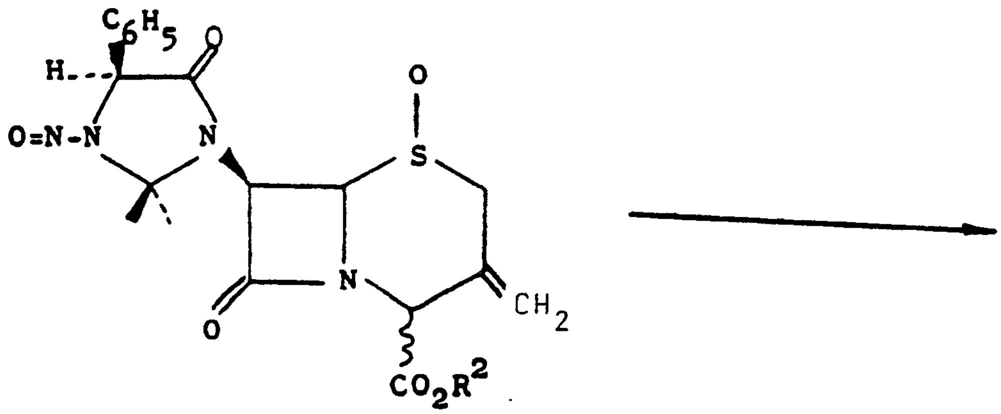

XIII

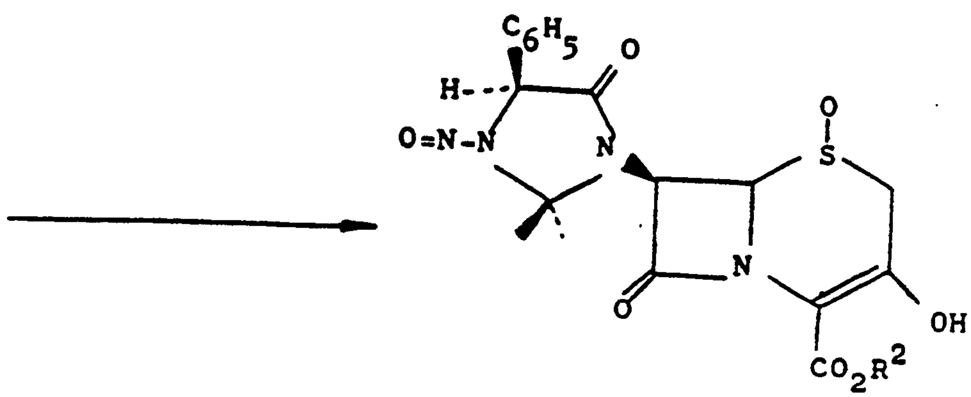

XIV

Stage 5

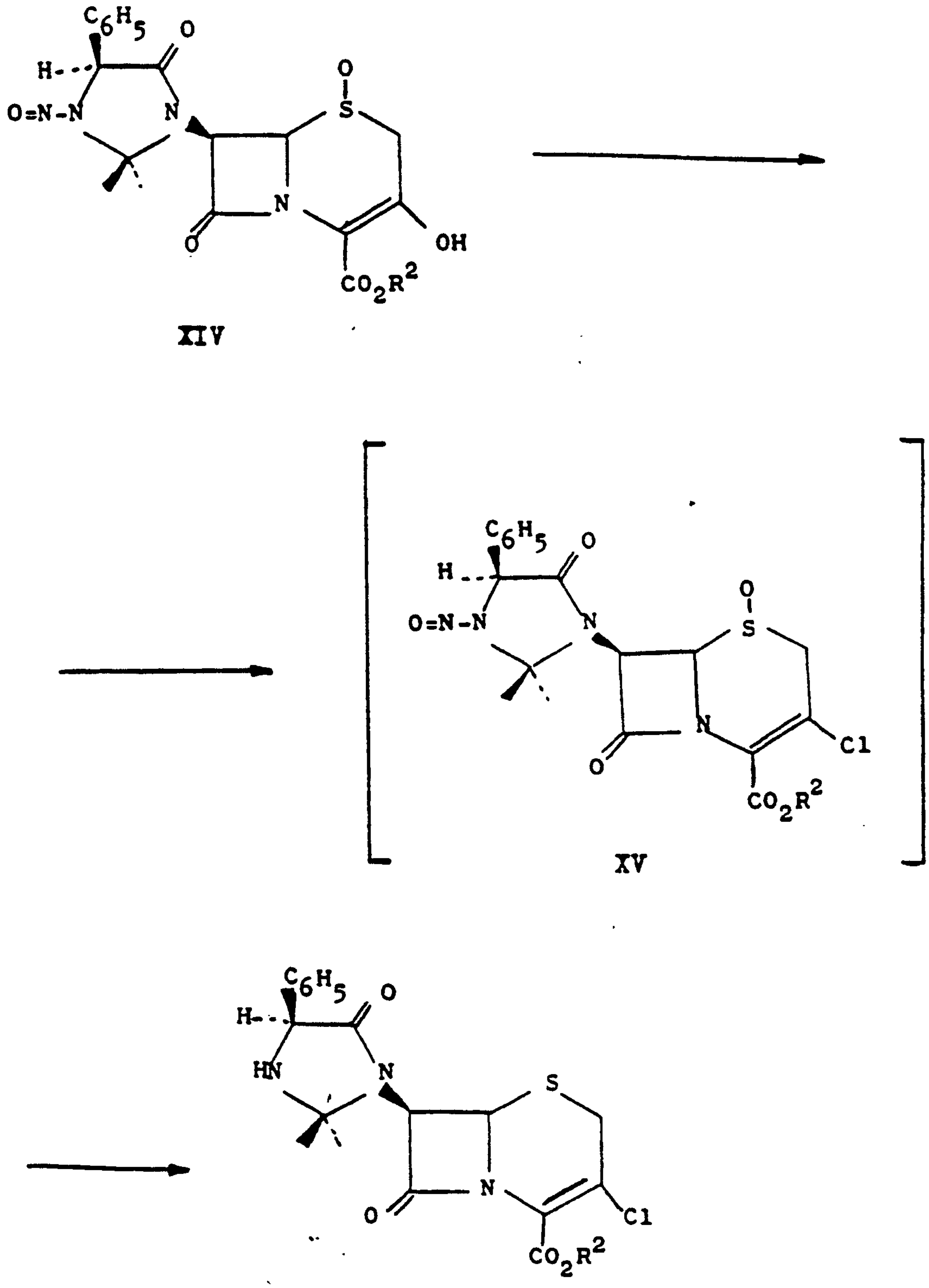

XIV

XV

XVI

Stage 6

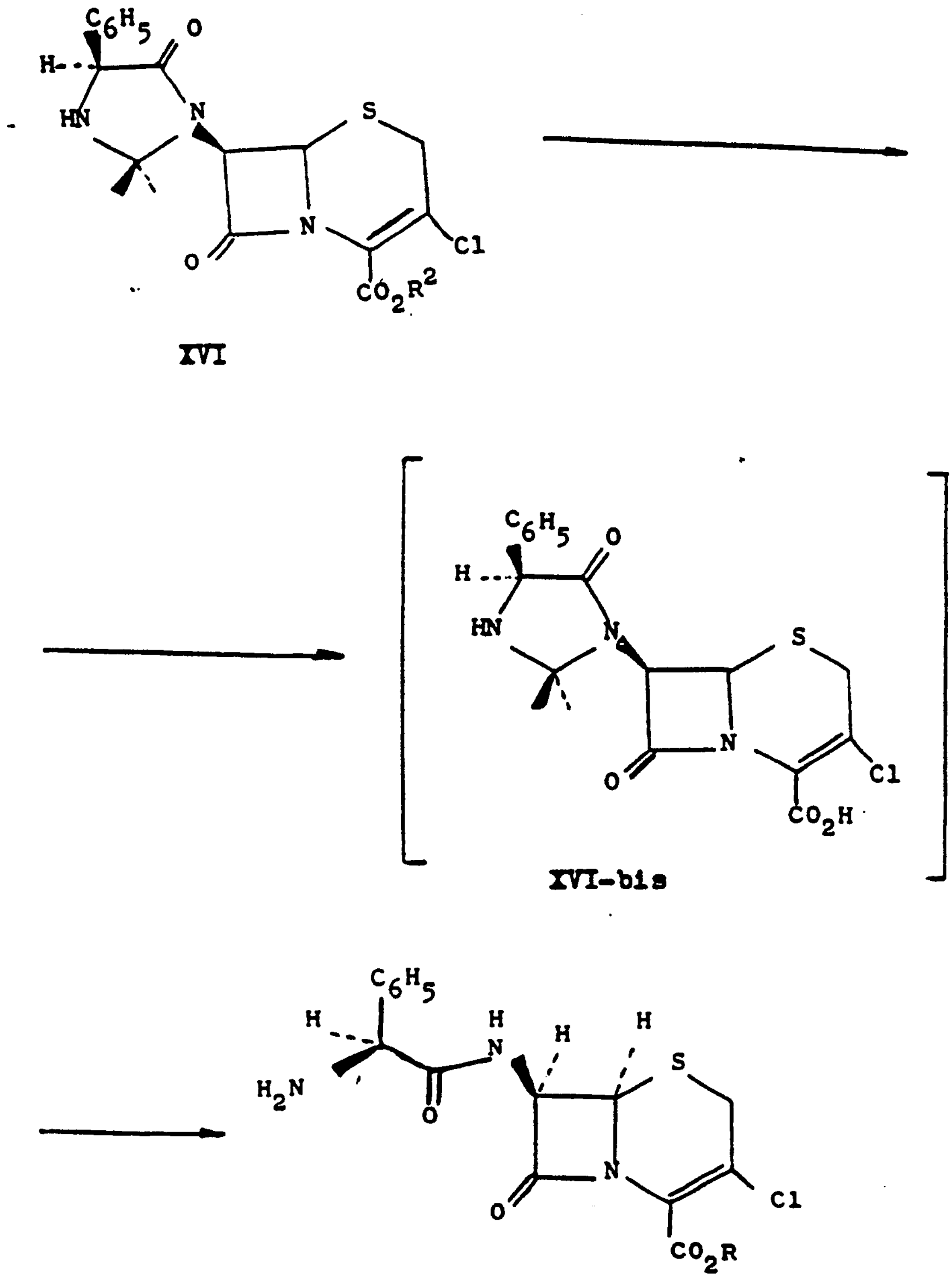

XVI

XVI-bis

I

This scheme should not be taken as being exclusive, but rather explanatory, since the order of any chemical stages can be varied without affecting the general process, for example in Stage 2, the order in which the reactions of esterification and oxidation are performed is immaterial to the end result.

The intermediate products in question are indicated in square brackets in accordance with the general notation of organic chemistry. The fact that they are not separated should not be interpreted as meaning that they cannot be separated, but that it is not necessary to do it and so the process becomes simpler by including various steps in a single stage.

Examples to illustrate in detail the procedure which forms the subject of this invention are given below. These examples should not be taken as being a limitation of the scope of the patent, but as typical experimental cases of the general procedure.

## EXAMPLE 1

(6R,7R)-7-[(R)-2-AMINO-2-PHENYLACETAMIDO]-3-CHLORO-8-OXO-5-THIA-1-AZABICYCLO[4,2,0]OCT-2-ENE-2-CARBOXYLIC ACID.

1. (5R,6R,4′R)-6-(2′,2′-Dimethyl-3-nitroso-5′-oxo-4′-phenyl-imidazolidin-1′-yl)-3,3-dimethyl-7-oxo-4-thia-1-azabicyclo[3,2,0]heptane-2-carboxylic acid.

2.6 ml (18.6 mmol) of triethylamine are added to a suspension of 3.5 g (10 mmol) of ampicillin in 15 ml of acetone and stirring takes place for 20 hours, at room temperature. After evaporating the solvent, 30 ml of water, 0.83 g (12 mmol) of sodium nitrite and 20 ml of ethyl acetate are added. The mixture is cooled in an ice bath and is acidified with hydrochloric acid to a pH of 2. The aqueous phase is decanted and the organic phase is washed successively with water and saturated sodium chloride solution, dried over anhydrous magnesium sulphate and evaporated to dryness. A white solid is obtained which corresponds to the title compound. It crystallizes from methanol/water. M.p. 167-169° C.

IR (Nujol) $\nu$ = 1800 (C=O $\beta$-lactam), 1740 ($CO_2H$), 1720 (C=O imidazolidine) 1440 (N-N=O) and 1090 $cm^{-1}$ (N-N=O) $^1H$-NMR (DMSO-$d_6$) $\delta$ = 13.3 (br.s., 1H, -$CO_2H$, interchanges $D_2O$), 7.3 (m, 5H, $C_6H_5$), 5.65 (S, 1H, CH-$C_6H_5$), 5.6 (d, J=4.5 Hz, 1H, H-6), 5.4 (d, J=4.5 Hz, 1H, H-5), 4.35 (s, 1H, H-2), 2.05 (s, 3H, $CH_3$ imidazoline), 2.0 (s, 3H, $CH_3$ imidazoline), 1.5 (s, 3H, $CH_3$ $\beta$) and 1.4 ppm (s, 3H, $CH_3$ $\alpha$)

UV (MeOH) $\lambda$ max. = 205 and 218 nm.

2. p-Nitrobenzyl (5R,6R,4′R)-6-(2′,2′- dimethyl-3′-nitroso-5′-oxo-4′-phenyl-imidazolidin-1′-yl)-3,3-dimethyl-7oxo-4thia-1-azabicyclo[3,2,0]heptane-2-carboxylate.

A suspension of 4.2 g (10 mmol) of (5R,6R,4′R)-6-(2′,2′-dimethyl-3′-nitroso-5′-oxo-4′-phenyl-imidazolidin-1′-yl)-3,3-dimethyl-7-oxo-4-thia-1-azabicyclo[3,2,0]heptane-2-carboxylic acid in a mixture of 50 ml of N,N-dimethylformamide and 40 ml of dioxan is treated with 0.9 g (10.7 mmol) of sodium bicarbonate and 2.4 g (11 mmol) of p-nitrobenzyl bromide for 24 hours, at room temperature. Next, 50 ml of water and 100 ml of ethyl acetate are added. The organic phase is decanted, and is washed with water and is dried over anhydrous magnesium sulphate. A solid is obtained by evaporation to dryness, which is crystallized from isopropanol and the analytic data of which correspond to the title product. M.p. 97-99° C.

IR (Nujol) $\nu$ = 1800 (C=O $\beta$-lactam), 1740 (C=O imidazolidinyl), 1700 ($CO_2R$), 1510, 1320 ($NO_2$), 1460 (N-N=O) and 1080 $cm^{-1}$ (N-N=O).

$^1H$-NMR ($CDCl_3$) $\delta$ = 7.9 (q, 4H, $C_6H_4$), 7.25 (m, 5H, $C_6H_5$), 5.65 (d, J=4 Hz, 1H, H-6), 5.45 (s, 1H, -CH-$C_6H_5$), 5.25 (s, 2H, $CH_2$), 4.95 (d, J=4 Hz, 1H, H-5), 4.6 (s, 1H, H-2), 2.1 (s, 6H, $2CH_3$ imidazoline), 1.6 (s, 3H, $CH_3$ $\beta$) and 1.4 ppm (s, 3H, $CH_3$ $\alpha$).

UV ($CHCl_3$) $\lambda$ max. = 235 and 260 nm.

3. p-Nitrobenzyl (4R,5R,6R,4′R)-6-(2′,2-dimethyl-3′-nitroso-5′-oxo-4′-phenyl-imidazolidin-1′-yl)-3,3-dimethyl-7-oxo-4-thia-1-azabicyclo[3,2,0]heptane-2-carboxylate 4-oxide.

An ozone current is passed through a solution of 5 g of p-nitrobenzyl (5R,6R,4′R)-6-(2′,2′-dimethyl-3′-nitroso-5′-oxo-4′-phenyl-imidazolidin-1′-yl)-3,3-dimethyl-7-oxo-4-thia-1-azabicyclo[3,2,0]heptane-2-carboxylate in 100 ml of acetone for 45 minutes, while it is cooled in a bath of solid carbon dioxide. The title product is obtained in the form of a white solid by evaporation to dryness. M.p. 119-121 °C.
IR (Nujol) $\nu$ = 1800 (C=O $\beta$ lactam), 1750 (C=O) imidazolidinyl), 1710 ($CO_2R$), 1520, 1340 ($NO_2$), 1470 (N-N=O) and 1050 $xm^{-1}$ (SO).
$^1$H-NMR ($CDCl_3$) $\delta$ = 7.9 (q, 4H, $C_6H_4$), 7.35 (m, 5H, $C_6H_5$), 5.5 (s, 1H, CH-$C_6H_5$), 5.3 (s, 2H, $CH_2$) 5.1 (d, J=4 Hz, 1H, H-6), 4.8 (d, J=4 Hz, 1H, H-5), 4.55 (s, 1H, H-2), 2.2 (s, 3H, $CH_3$ imidazoline), 2.1 (s, 3H, $CH_3$ imidazoline), 1.6 (s, 3H, $CH_3$ $\beta$) and 1.2 ppm (s, 3H, $CH_3$ $\alpha$)
UV ($CHCl_3$) $\lambda$ max. = 258 nm.

4. p-Nitrobenzyl (6R,7R,4′R)-7-(2′,2′-dimethyl-3′-nitroso-5′-oxo-4′-phenyl-imidazolidin-1′-yl)-3-exomethylen-8-oxo-5-thia-1-azabicyclo[4,2,0]octane-2-carboxylate 5-oxide.

A mixture of 5.7 g (10 mmol) of p-nitrobenzyl (4R,5R,6R,4′R)-6-(2′,2′-dimethyl-3′-nitroso-5′-oxo-4′-phenyl-imidazolidin-1′-yl)-3,3-dimethyl-7-oxo-4-thia- 1-azabicyclo[3,2,0]heptane-2-carboxylate 4-oxide, 200 ml of dry chloroform, 3.5 ml (50 mmol) of propylene oxide and 3 g (22 mmol) of N-chlorosuccinimide is stirred under reflux for 6 hours under dry nitrogen. Half the solvent is removed, and the mixture is cooled to precipitate the succinimide which is filtered off. The filtrate is evaporated to dryness and the residue obtained is dissolved in 40 ml of dry toluene. Next, a solution of 2.35 ml (20 mmol) of tin tetrachloride in 5 ml of dry toluene is slowly added and the mixture is stirred for 14 hours, at room temperature. The precipitate formed is filtered and washed successively with toluene and pentane. The solid obtained is suspended in 180 ml of cold ether and is stirred for 30 minutes. The suspension is filtered and precipitated from ethanol as a mixture of diastereoisomers of the title compound, which are separated by high-pressure liquid chromatography, using a mixture of ethyl acetate - hexane in a 2 : 1 proportion as an eluent.

The 5R sulphoxide is separated as a colourless foam. IR (Nujol) $\nu$ = 1780 (C=O $\beta$-lactam), 1720 (C=O imidazolidinyl), 1700 ($CO_2R$), 1520 and 1350 $cm^{-1}$ ($NO_2$).
$^1$H-NMR ($CDCl_3$) $\delta$ = 7.9 (q, 4H, $C_6H_4$), 7.3 (m, 5H, $C_6H_5$), 5.7 (s, 1H, CH-$C_6H_5$), 5.6 (d, 1H, J=4.5 Hz, H-7), 5.35 (s, 2H, $CH_2$), 5.3 (s, 2H, =$CH_2$), 5.1 (d, 1H, J=4.5 Hz, H-6), 3.5 (q, 1H, H-2) and 2.3 ppm (s, 6H, 2CH3 imidazoline) UV ($CHCl_3$) $\lambda$ max. = 235 and 260 nm.

The 55 sulphoxide is separated in the form of a foam. IR (Nujol) $\nu$ = 1780 (C=O $\beta$-lactam), 1720 (C=O imidazolidinyl), 1700 ($CO_2R$), 1520 and 1350 $cm^{-1}$ ($NO_2$).
$^1$H-NMR ($CDCl_3$) $\delta$ = 7.9 (q, 4H, $C_6H_4$), 7.3 (m, 5H, $C_6H_5$), 5.5 (s, 1H, CH-$C_5H_5$), 5.45 (s, 1H, =$CH_2$), 5.35 (s, 2H, $CH_2$), 5.25 (s, 1H, =$CH_2$), 5.2 (d, J=4.5 Hz, 1H, H-7), 4.8 (d, J=4.5 Hz, 1H, H-6), 3.5 (q, 1H, H-2), 2.15 (s, 3H, $CH_3$ imidazoline) and 2.05 ppm (s, 3H, $CH_3$ imidazoline)
UV ($CHCl_3$) $\lambda$ max. = 235 and 260 nm.

5. p-Nitrobenzyl (6R,7R,4′R)-7-(2′,2′- dimethyl-3′-nitroso-5′-oxo-4′-phenyl-imidazolidin-1′- yl)-3-hydroxy-8-oxo5-thia-1-azabicyclo[4,2,0]oct-2-ene-2-carboxylate 5-oxide.

A solution of 1 g (1.7 mmol) of p-nitrobenzyl (6R,7R,4′R)-7-(2′,2′-dimethyl-3′-nitroso-5′-oxo-4′-phenyl-imidazolidin-1′-yl)-3-exomethylen-8-oxo-5-thia-1-azabicyclo[4,2,0]octane-2-carboxylate 5-oxide in 180 ml of methylene chloride is cooled in a bath of solid carbon dioxide and an ozone current is passed until blue coloration occurs. An oxygen current is then passed through for some minutes in order to remove the excess ozone and evaporation then takes place, so as to produce the product of the heading in the form of a white foam as a mixture of diastereoisomers in the form of a white foam.
IR (Nujol) $\nu$ = 1780 (C=O $\beta$-lactam), 1720 (C=O imidazolidinyl), 1700 ($CO_2R$) and 1200 $cm^{-1}$ (a, OH).
$^1$H-NMR ($CDCl_3$) $\delta$ = 7.9 (q, 4H, $C_6H_4$), 7.3 (m, 5H, $C_6H_5$), 5.6 and 5.5 (s, 1H, CH-$C_6H_5$), 5.4 (s, 2H, $CH_2$) 5.1 and 5.05 (d, J=4.5 Hz, 1H, H-7), 4.8 and 4.6 (d, J=4.5 Hz, 1H, H-6), 4.0 and 3.6 (g, 2H, H-2), 2.2 (a, OH, interchanges $D_2O$), 2.3, 2.25, 2.2 and 2.15 ppm (s, 6H, 2$CH_3$ imidazoline).

6. p-Nitrobenzyl (6R,7R,4′R)-7-(2′,2′-dimethyl-5′-oxo-4′-phenyl-imidazolidin-1′-yl)-3-chloro-8-oxo-1-aza-5-thiabicyclo[4,2,0]oct-2-ene-2-carboxylate.

A solution of 1.2 g (2 mmol) of p-nitrobenzyl (6R,7R,4′R)-7-(2′,2′-dimethyl-3′-nitroso-5′-oxo- 4′-phenyl-imidazolidin-1′-yl)-3-hydroxy-8-oxo-1-aza-5-thiabicyclo [4,2,0]oct-2-ene-2-carboxylate 5-oxide in 10 ml of dry N,N-dimethylformamide is cooled in a bath of solid carbon dioxide and is treated with a solution of 0.32 ml (4 mmol) of phosphorus trichloride in 4 ml of dry N,N-dimethylformamide. It is stirred for 1 hour below 0° C and for 1 hour at room temperature. The reaction mixture is poured over ice water and the precipitate formed is filtered, washed with water several times and dried in a vacuum, yielding the product of the heading in the form of a foam.
IR (Nujol) $\nu$ = 1800 (C=O $\beta$-lactam), 1740 (C=O imidazolidinyl), 1720 $cm^{-1}$ ($CO_2R$).
$^1$H-NMR ($CD_3OD$) $\delta$ =7.9 (q, 4H, $C_6H_4$), 7.4 (m, 5H, $C_6H_5$), 5.4 (d, J=4.5 Hz, 1H, H-7), 5.2 (s, 1H, CH-$C_6H_5$), 5.35 (s, 2H, $CH_2$), 5 (d, J=4.5 Hz, 1H, H-6), 3.75 (q, 2H, H-2), 1.55 (s, 3H, $CH_3$ imidazoline) and 1.45 ppm (s, 3H, $CH_3$ imidazoline).

7. (6R,7R)-7-[(R)-2-Amino-2-phenylacetamido]-3-chloro-8-oxo-5-thia-1-azabicyclo[4,2.0]oct2-ene-2-carboxylic acid.

1.5 ml of trifluoroacetic acid is added to a solution of 0.54 g (1 mmol) of p-nitrobenzyl (6R,7R,4′R)-7-(2′,2′-dimethyl-5-oxo-4′-phenyl-imidazolidin-1′-yl)-3-chloro-8-oxo-5-thia-1-azabicyclo[4,2,0]oct-2-ene-2-carboxylate in 1 ml of anisole cooled to 0° C, and stirring takes place for 30 minutes between 5 and 10° C. 50 ml of ether is added and the precipitate obtained is filtered, washed with ether and dried in a vacuum. This solid is treated in an aqueous medium. which is acidified at a pH of 4.5 with dilute hydrochloric acid, thereby precipitating the title product in the form of a hydrochloride. IR (Nujol) $\nu$ = 1780 (C=O $\beta$-lactam). 1690 (C=O amide), 1600 ($CO_2H$) and 697 $cm^{-1}$ (C-Cl).
$^1$H-NMR ($D_2O$+Cl) $\delta$ = 7.6 (s, 5H, $C_6H_5$), 5.8 (d, J=5 Hz, 1H, H-7), 5.3 (s, 1H, CH-$C_6H_5$), 5.2 (d, J=5 Hz. 1H, H-6) and 3.7 ppm (q, 2H, H-2).
UV (HCl 0.1 M) λ max. = 230 and 265 nm.

## EXAMPLE 2

P-NITROBENZYL (5R,6R,4′R)-6-(2′,2′-DIMETHYL-3′-NITROSO-5′-OXO-4′-PHENYL-IMIDAZOLIDIN-1′-YL)-3,3-DIMETHYL-7-OXO-4-THIA-1-AZABICYCLO[3,2,0]HEPTANE-2-CARBOXYLATE 4-OXIDE (XI).

0.86 g (5 mmol) of m-chloroperbenzoic acid is added to a solution of 1.1 g (2 mmol) of p-nitrobenzyl (5R,6R,4′R)-6-(2′,2′-dimethyl-3′-nitroso-5′-oxo-4′-phenyl-imidazolidin-1′-yl)-3,3-dimethyl-7-oxo-1-aza-4-thiabicyclo [3,2,0]heptane-2-carboxylate in 20 ml of methylene chloride, while cooling takes place in an ice bath. Stirring takes place for 3 more hours between 5 and 10° C, at the end of which the precipitate formed is filtered off and the filtrate is washed successively with water, a saturated sodium bicarbonate solution and a saturated sodium chloride solution. The organic phase is dried over anhydrous magnesium sulphate and by subsequent evaporation a white solid is obtained which corresponds to the compound in the heading.

## EXAMPLE 3

METHYL (5R,6R,4′R)-6-(2′,2′-DIMETHYL-3′-NITROSO-5′-OXO-4′-PHENYL-IMIDAZOLIDIN-1′-YL)-3,3-DIMETHYL-7-OXO-4-THIA-1-AZABICYCLO[3,2,0]HEPTANE-2-CARBOXYLATE (X).

A suspension of 4.2 g (10 mmol) of (5R,6R,4′R)-6-(2′,2′-dimethyl-3′-nitroso-5′-oxo-4′-phenyl-imidazolidin-1′-yl)-3,3-dimethyl-7-oxo-4-thia-1-azabicyclo[3,2,0]heptane-2-carboxylic acid, 0.9 g (10.7 mmol of sodium bicarbonate and 1.3 ml (21 mmol) of methyl iodide in 50 ml of N,N-dimethylformamide is stirred at room temperature for 24 hours, at the end of which it is poured over crushed ice. The white precipitate formed is washed with water and is dried in a vacuum. The said solid which corresponds to the title product is crystallized from methanol. M.p. 179-181° C.
IR (Nujol) $\nu$ = 1790 (C=O $\beta$-lactam), 1740 (C=O imidazolidinyl), 1720 ($CO_2H$), 1440 (N-N=O) and 1090 $cm^{-1}$ (N-N=O).

$^1$H-NMR ($CDCl_3$) $\delta$ = 7.3 (m, 5H, $C_6H_5$), 5.56 (d, 1H, J=4 Hz, H-5), 5.45 (sa, 1H, CH-$C_6H_5$), 4.95 (da, J=4 Hz, H-6), 4.55 (s, 1H, H-3), 3.8 (s, 3H, $CO_2CH_3$), 2.10 (s, 6H, $CH_3$ imidazoline), 1.65 (s, 3H, $CH_3$ $\beta$) and 1.5 ppm (s, 3H, $CH_3$- $\alpha$)
UV ($Cl_3CH$) $\lambda$ max. = 242 nm.

## EXAMPLE 4

METHYL (4R,5R,6R,4′R)-6-(2′,2′-DIMETHYL-3′-NITROSO-5′-OXO-4′-PHENYL-IMIDAZOLIDIN-1′-YL)-3,3-DIMETHYL-7-OXO-4-THIA-1-AZABICYCLO[3,2,0]HEPTANE-2-CARBOXYLATE 4-OXIDE (XI).

A current of ozone is passed for 30 minutes over a solution of 4.3 g (10 mmol) of methyl (5R,6R,4′R)-6-(2′,2′-dimethyl-3′-nitroso-5′-oxo-4′-phenyl-imidazolidin-1′-yl)-3,3-dimethyl-7-oxo-4-thia-1-azabicyclo[3,2,0]-heptane-2-carboxylate in 100 ml of methylene chloride, while cooling takes place in a bath of of solid carbon dioxide. The product of the heading is obtained by subsequent evaporation. M.p. 129-131 °C.

IR (Nujol) $\nu$ = 1800 (C=O $\beta$-lactam), 1740 (C=O imidazolidinyl), 1720 ($CO_2R$), 1440 (N-N=O) and 1060 $cm^{-1}$ (SO).
$^1$H-NMR ($CDCl_3$) = 7.3 (m, 5H, $C_6H_5$), 5.5 (s, 1H, CH-$C_6H_5$), 5.1 (d, J=4 Hz, 1H, H-6), 4.75 (d, J=4 Hz 1H, H-5), 4.5 (s, 1H, H-2), 3.8 (s, 3H, $CO_2CH_3$), 2.2 (s, 6H, $2CH_3$ imidazolidine), 1.6 (s, 3H, $CH_3$ $\beta$) and 1.2 ppm (s, 3H. $CH_3$ $\alpha$)
UV ($CHCl_3$) $\lambda$ max. = 242 nm.

## EXAMPLE 5

METHYL (5R,6R,4′R)-6-(2′,2′-DIMETHYL-3′-NITROSO- 5′-OXO-4′-PHENYL-IMIDAZOLIDIN-1′-YL)-3,3-DIMETHYL-7-OXO-4-THIA-1-AZABICYCLO[3,2,0]HEPTANE-2-CARBOXYLATE 4-OXIDE (XI).

A solution of 0.43 g (1 mmol) of methyl (5R,6R,4′R)-6-(2′,2′-dimethyl-3′-nitroso-5′-oxo-4′-phenyl-imidazolidin-1′-yl)-3,3-dimethyl-7-oxo-4-thia-1-azabicyclo[3,2,0]heptane-2-carboxylate in 5 ml of methylene chloride is cooled in a bath of solid carbon dioxide and 0.6 ml of peracetic acid is added slowly up to 40% Stirring takes place for 3 hours at room temperature, at the end of which it is washed successively with water and saturated sodium chloride solution. After drying over anhydrous magnesium sulphate and evaporation of the solvent, the product of the heading is obtained in solid form.

## EXAMPLE 6

P-NITROBENZYL (5R,6R)-6-(2′,2′-DIMETHYL-3′-NITROSO-5′-OXO-4′-PHENYL-IMIDAZOLIDIN-1′-YL)-3,3-DIMETHYL-7-OXO-4-THIA-1-AZABICYCLO[3,2,0]HEPTANE-2-CARBOXYLATE. (X)

A mixture of 4.2 g (10 mmol) of (5R,6R,4′R)-6-(2′,2′-dimethyl-3′-nitroso-5′-oxo-4′-phenyl-imidazolidin-1′-yl)-3,3-dimethyl-7-oxo-4-thia-1-azabicyclo[3,2,0]heptane-2-carboxylic acid, 0.9 g (11 mmol) of sodium bicarbonate, 3.4 g (11 mmol) of benzyltributylammonium chloride, 2.16 g (10 mmol) of p-nitrobenzyl bromide and 42 ml of acetonitrile are stirred at room temperature for 1 hour. Then 50 ml of water are added and extraction is carried out with ethyl acetate. The extracts are washed successively with water and a saturated sodium chloride solution. Drying over anhydrous magnesium sulphate and subsequent evaporation are carried out, thereby obtaining the title product in the form of a solid.

## EXAMPLE 7

(4R,5R,6R,4′R)-6-(2′,2′-DIMETHYL-3′-NITROSO-3′-OXO-4′-PHENYL-IMIDAZOLIDIN-1′-YL)-3,3-DIMETHYL-7-OXO-4-THIA-1-AZABICYCLO[3,2,0]HEPTANE-2-CARBOXYLIC ACID 4-OXIDE. (XIa)

A solution of 0.5 g (1.2 mmol) of (5R,6R,4′R)-6-(2′,2′-dimethyl-3′-nitroso-5′-oxo-4′-phenyl-imidazolidin-1′-yl)-3,3-dimethyl-7-oxo-4-thia-1-azabicyclo[3,2,0]heptane-2-carboxylic acid in a mixture of 5 ml of acetone and 25 ml of water, adjusted to a pH of 2.7, is cooled in a bath of ice and a current of ozone is passed through for 2 hours. The solvent is evaporated and the aqueous solution is acidified with dilute sulphuric acid to a pH of 2. The precipitate formed is filtered, washed with water and dried in a vacuum, yielding the title product in the form of a white solid. M.p. 160 °C (dec).
IR (Nujol) $\nu$ = 1800 (C=O $\beta$-lactam), 1740 (C=O imidazolidinyl), 1720 ($CO_2H$), 1450 (N-N=O), 1080 (N-N=O) and 1050 $cm^{-1}$ (SO).
$^1H$-NMR (DMSO-$d_6$) $\delta$ = 7.32 (m, 5H, $C_6H_5$), 5.77 (s, 1H, CH-$C_6H_5$), 5.72 (d, J=5 Hz, 1H, H-6), 4.83 (d, J=5 Hz, 1H, H-5), 4.30 (s, 1H, H-2), 2.12 (s, 3H, $CH_3$ imidazolidine), 2.05 (s, 3H, $CH_3$ imidazolidine), 1.47 (s, 3H, $CH_3$ $\beta$) and 1.20 ppm (s, 3H, $CH_3$ $\alpha$)
UV (MeOH) $\lambda$ max. = 205 and 218 nm.

## EXAMPLE 8

P-NITROBENZYL (4R,5R,6R,4′R)-6-(2′,2′-DIMETHYL-3′-NITROSO-5′-OXO-4′-PHENYL-IMIDAZOLIDIN-1′-YL)-3,3-DIMETHYL-7-OXO-4-THIA-1-AZABICYCLO[3,2,0]HEPTANE-2-CARBOXYLATE 4-OXIDE.

A solution of 5 g (11 mmol) of (4R,5R,6R,4′R)-6-(2′,2′-dimethyl-3′-nitroso-5′-oxo-4′-phenyl-imidazolidin-1′-yl)-3,3-dimethyl-7-oxo-4-thia-1-azabicyclo[3,2,0]heptane-2-carboxylic acid 4-oxide in 100 ml of N,N-dimethylformamide and 40 ml of dioxane is treated with 3 g (14 ml) of p-nitrobenzyl bromide and 1.2 g (14 mmol) of sodium bicarbonate, with stirring, for 4 hours at room temperature. Next, it is poured over ice water and the precipitate formed is filtered off, which corresponds to the title product.

## EXAMPLE 9

METHYL (4R,5R,6R,4′R)-6-(2′,2′-DIMETHYL-3′-NITROSO-5′-OXO-4′-PHENYL-IMIDAZOLIDIN-1′-YL)-3,3-DIMETHYL-7-OXO-4-THIA-1-AZABICYCLO[3,2,0]HEPTANE-2-CARBOXYLATE 4-OXIDE (XI).

5 g (11 mmol) of (4R, 5R, 6R,4′R)-6-(2′,2′-dimethyl-3′-nitroso-5′-oxo-4′-phenyl-imidazolidin-1-yl)-3,3dimethyl-7-oxo-4-thia-1-azabicyclo[3,2,0]heptane-2- carboxylic acid 4-oxide are reacted with 1 ml (16 mmol) of methyl iodide, in accordance with the procedure described in Example 5, thereby yielding the title product.

## EXAMPLE 10

METHYL (6R,7R,4′R)-6-(2′,2′-DIMETHYL-3′-NITROSO- 5′-OXO-4′-PHENYL-IMIDAZOLIDIN-1′-YL)-3-EXOMETHYLEN-8-OXO-5-THIA-1-AZABICYCLO[4,2,0]OCTANE-2-CARBOXYLATE 5-OXIDE (XIII).

A mixture of 2.5 g (5.5 mmol) of methyl (4R,5R,6R,4′R)-6-(2′,2′-dimethyl-3′nitroso-5′-oxo-4′-phenyl-imidazolidin-1′-yl)-3,3-dimethyl-7-oxo-1-aza-4-thiabicyclo[3,2,0]heptane-2-carboxylate 4-oxide, 1.5 g (11 mmol) of N-chlorosuccinimide and 1.8 ml (26 mmol) of propylene oxide in 180 ml of dry carbon tetrachloride is heated between 60 and 80 °C for 2 hours under nitrogen. The solvent is evaporated and the residue obtained is dissolved in 50 ml of dry benzene and is cooled in an ice bath. The precipitate formed is filtered off and a solution of 1.5 ml (12 mmol) of tin tetrachloride in 5 ml of dry benzene is added onto the filtrate which is then stirred for 14 hours at room temperature. The precipitate formed is filtered off and

washed with benzene. Once it is dry it is stirred with 25 ml of methanol in an ice bath for 5 hours. The solvent is evaporated and 50 ml of ethyl acetate and 50 ml. of water are added. The organic phase is decanted, and is dried over anhydrous magnesium sulphate and is subsequently evaporated, yielding the product of the heading as a mixture of diastereoisomers, which are separated by high-pressure liquid chromatography, using an ethyl acetate - hexane mixture in a 2 : 1 proportion as an eluent.

The isomer 55 is obtained in the form of a foam. IR (Nujol) $\nu$ = 1790 (C=O $\beta$-lactam), 1740 (C=O imidazolidinyl) and 1720 $cm^{-1}$ ($CO_2R$).

$^1$H-NMR ($CDCl_3$) $\delta$ = 7.3 (m, 5H, $C_6H_5$), 5.7 (sa, 1H, =$CH_2$), 5.55 (sa, 1H, $\overline{CH}$-$C_6H_5$), 5.45 (sa, 1H, =$CH_2$), 5.2 (s, 1H, H-4), 5.05 (m, 2H, H-6 and H-7), 3.8 (s, 3H, $CO_2CH_3$), 3.7 (q, $\overline{2H}$, H-2) and 2.25 ppm (s, 6H, $2CH_3$ imidazolidine)

UV ($Cl_3$) $\lambda$ max. = 232 and 240 nm.

The isomer 5R is separated as a colourless foam. IR (Nujol) $\nu$ = 1790 (C=O $\beta$-lactam), 1740 (C=O imidazolidinyl) and 1720 $cm^{-1}$ ($CO_2R$).

$^1$H-NMR ($CDCl_3$) = 7.3 (m, 5H, $C_6H_5$), 5.5 (s, 1H, $\overline{CH}$-$C_6H_5$), 5.45 (s, 1H, =$CH_2$), 5.4 (s, 1H, =$CH_2$), 5.1 (s, 1H, H-4), 5.05 (d, J=4.5 Hz, 1H, H-7), 4.8 (d, J=4.5 Hz, 1H, H-6), 3.65 (q, 2H, H-2), 2.15 (s, 3H, $CH_3$ imidazolidine) and 2.05 ppm (s, 3H, $CH_3$ imidazolidine)

UV ($Cl_3$) $\lambda$ max. = 232 and 240 nm.

## EXAMPLE 11

P-NITROBENZYL (6R,7R,4′R)-7-(2′,2′-DIMETHYL-3′-NITROSO-5′-OXO-4′-PHENYL-IMIDAZOLIDIN-1′-YL)-3-EXOMETHYLEN-8-OXO-5-THIA-1-AZABICYCLO[4,2,0]HEPTANE-2-CARBOXYLATE 5-OXIDE (XIII).

A suspension of 4 g (7 mmol) of p-nitrobenzyl (4R,5R,6R,4′R)-6-(2′,2′-dimethyl-3′-nitroso-5′-oxo-4′-phenyl-imidazolidin-1′-yl)-3,3-dimethyl-7-oxo-4-thia-1-azabicyclo[3,2,0]heptane-2-carboxylate 4-oxide in 150 ml of 1,2-dichloroethane is treated with 2.7 g (20 mmol) of N-chlorosuccinimide and 3 ml of propylene oxide between 60 and 80 °C for 4 hours under nitrogen. The mixture is concentrated to 1/5 of the total volume and cooled in an ice bath. The succinimide is filtered off and the solvent is evaporated. The residue obtained is dissolved in a mixture of 100 ml of toluene and 10 ml of ether and, cooling in an ice bath, a solution of 1.6 ml (14 mmol) of tin tetrachloride in 10 ml of toluene is added. The mixture is stirred for 20 hours at room temperature, at the end of which the precipitate formed is filtered off and washed with toluene. Once it is dry it is stirred in an ice bath together with 100 ml of isopropanol for 1 hour, precipitating the title product, as a mixture of the R and S sulphoxides, which can be separated by high-pressure liquid chromatography, using ethyl acetate -hexane mixture in a 2 : 1 proportion as an eluent.

## EXAMPLE 12

METHYL (6R,7R,4′R)-7-(2′,2′-DIMETHYL-3′-NITROSO-5′-OXO-4′-PHENYL-IMIDAZOLIDIN-1′-YL)-3-EXOMETHYLEN-8-OXO-5-THIA-1-AZABICYCLO[4,2,0]OCTANE-2-CARBOXYLATE 5-OXIDE. (XIII)

35 ml (50 mmol) of propylene oxide and 400 ml of dry toluene are added to a mixture of 5 g (11 mmol) of methyl (4R,5R,6R,4′R)-6-(2′,2′-dimethyl-3′-nitroso-5′-oxo-4′-phenyl-imidazolidin-1′-yl)-3,3-dimethyl-7-oxo-4-thia-1-azabicyclo[3,2,0]heptane-2-carboxylate 4-oxide and 4 g (22 mmol) of N-chlorophthalimide. The mixture is heated to 70-80 °C for 3 hours under nitrogen, and then left to reach room temperature, thereby causing phthalimide to precipitate, which is filtered off. The filtrate is added into a solution of 2.8 ml (23 mmol) of tin tetrachloride in 10 ml of dry toluene, while it is cooled in an ice bath. It is stirred for 15 hours at room temperature.

After this final process, the product of the heading is obtained, as a mixture of the R and S sulphoxides which can be separated by high-pressure liquid chromatography, using an ethyl acetate - hexane mixture in a 3 : 1 proportion as an eluent.

EXAMPLE 13

METHYL (5R,6R,7R,4′R)-7-(2′,2′-DIMETHYL-3′-NITROSO-5′-OXO-4′-PHENYL-IMIDAZOLIDIN-1′-YL)-3-HYDROXY-8-OXO-5-THIA-1-AZABICYCLO[4,2,0]OCT-2-ENE-2-CARBOXYLATE 5-OXIDE (XIV).

A mixture of 0.4 g (0.9 mmol) of methyl (5R,6R,7R,4′R)-7-(2′,2′-dimethyl-3′-nitroso-5′-oxo-4′-phenyl-imidazolidin-1′-yl)-3-exomethylen-8-oxo-5-thia-1-azabicyclo[4,2,0]octane-2-carboxylate 5-oxide in 80 ml of methylene chloride is cooled in a bath of solid carbon dioxide and an ozone current is passed through it until a blue colour appears. Next, sulphur dioxide is passed through for 2 minutes and the solvent is evaporated, yielding the title product in the form of a white foam.
IR (Nujol) $\nu$ = 3300 (OH), 1800 (C=O $\beta$-lactam), 1720 (C=O imidazolidine), 1710 ($CO_2R$) and 1060 $cm^{-1}$ (SO).
$^1H$-NMR ($CDCl_3$) $\delta$ = 7.3 (m, 5H, $C_6H_5$), 5.55 (s, 1H, CH-$C_6H_5$), 5.1 (d, J=4.5 Hz, 1H, H-7), 4.8 (d, J=4.5 Hz, 1H, H-6), 3.95 (s, 3H, $CO_2CH_3$), 3.8 (br.s., 1H, OH, interchanges $D_2O$), 3.7 (q, 2H, H-2), 2.28 (s, 3H, $CH_3$ imidazolidine) and 2.25 ppm (s, 3H, $CH_3$ imidazolidine).

EXAMPLE 14

METHYL (5R,6R,7R,4′R(-7-(2′,2′-DIMETHYL-3′-NITROSO-5′-OXO-4′-PHENYL-IMIDAZOLIDIN-1′-YL)-3-HYDROXY-8-OXO-5-THIA-1-AZABICYCLO[4,2,0]OCT-2-ENE-2-CARBOXYLATE 5-OXIDE (XIV).

A solution of 0.5 g (1.1 mmol) of methyl (5R,6R,7R,4′R)-7-(2′,2′-dimethyl-3′-nitroso-5′-oxo-4′-phenyl-imidazolidin-1′-yl)-3-exomethylen-8-oxo-5-thia-1-azabicyclo[4,2,0]octane-2-carboxylate 5-oxide in 50 ml of methylene chloride is cooled in a bath of solid carbon dioxide and an ozone current is passed through it for some minutes until a blue colour appears. Next, oxygen is passed through for 1-2 minutes and 0.3 g (2.9 mmol) of sodium bisulphite is added. The mixture is stirred in an ice bath until a reaction with potassium iodide is negative. The solid in suspension is filtered off and the filtrate is then evaporated. The title product is thereby obtained in the form of a white foam.
IR (Nujol) $\nu$ = 3300 (OH), 1800 (C=O $\beta$-lactam), 1740 (C=O imidazolidinyl), 1710 ($CO_2R$) and 1040 $cm^{-1}$ (SO).
$^1H$-NMR ($CDCl_3$) $\delta$ = 7.3 (m, 5H, $C_6H_5$), 5.45 (s, 1H, CH-$C_6H_5$), 5.05 (d, J=4.5 Hz, 1H, H-7), 4.6 (d, J=4.5 Hz, 1H, H-6), 3.9 (s, 3H, $CO_2CH_3$), 4.0 (q, 2H, H-2), 2.8 (sa, 1H, OH, interchanges $D_2O$), 2.15 (s, 3H, $CH_3$ imidazolidine) and 2.1 ppm (s, 3H, $CH_3$ imidazolidine).

EXAMPLE 15

P-NITROBENZYL (5S,6R,7R,4′R)-7-(2′,2′-DIMETHYL-3′-NITROSO-5′-OXO-4′-PHENYL-IMIDAZOLIDIN-1′-YL)-3-HYDROXY-8-OXO-5-THIA-1-AZABICYCLO[4,2,0]OCT-2-ENE-2-CARBOXYLATE 5-OXIDE (XIV).

A solution of 0.5 g (8.8 mmol) of p-nitrobenzyl (5S,6R,7R,4′R)-7-(2′,2′-dimethyl-3′-nitroso-5′-oxo-4′-phenyl-imidazolin-1′-yl)-3-exomethylen-8-oxo-5-thia-1-azabicyclo[4,2,0]octane-2-carboxylate 5-oxide in a mixture of 60 ml of methylene chloride and 10 ml of methanol is cooled in a bath of solid carbon dioxide and an ozone current is passed through it until a blue colour appears. Next, dry nitrogen is passed through for some minutes. A white solid, which corresponds to the title product, is obtained by evaporation of the solvent.

EXAMPLE 16

METHYL (6R,7R,4′R)-7-(2′-DIMETHYL-5′-OXO-4′-PHENYL-IMIDAZOLIDIN-1′-YL)-3-CHLORO-8-OXO-5-THIA-1-AZABICYCLO [4,2,0]OCT-2-ENE-2-CARBOXYLATE (XVI).

0.18 ml (2.2 mmol) of phosphorus trichloride is slowly added to a solution of 0.45 g (1 mmol) of methyl (5S,6R,7R,4′R)-7-(2′,2′-dimethyl-3-nitroso-5′-oxo-4′-phenyl-imidazolidin-1′-yl)-3-hydroxy-8-oxo-5-thia-azabicyclo[4,2,0]oct2-ene-2-carboxylate 5-oxide in 5 ml of dry N,N-dimethyl formamide in a bath of ice. The reaction mixture is stirred for 1 hour at low temperature and for 1 hour at room temperature. It is then poured over ice, precipitating a solid which is filtered off, washed with water and dilute hydrochloric acid and dried in a vacuum, yielding the title product.

Following the previous procedure, starting from methyl (5R,6R,7R,4′R)-7-(2′,2′-dimethyl-3′-nitroso-5′-oxo-4′-phenyl-imidazolidin-1′-yl)3-hydroxy-8-oxo-5-thia-1-azabicyclo[4,2,0]oct-2-ene-2-carboxylate 5-oxide, the product of the heading is obtained in the same way.

IR (Nujol) $\nu$ = 1790 (C=O $\beta$-lactam), 1740 (C=O imidazolidinyl), 1720 ($CO_2R$) and 740 $cm^{-1}$ (C-Cl).

$^1$H-NMR ($CDCl_3$) $\delta$ = 7.4 (sa, 1H, interchanges $D_2O$, NH), 5.55 (sa, 1H, interchanges $D_2O$, CH-$C_6H_5$), 5.15 (d, J=4.5 Hz, 1H, H-7), 4.85 (d, J=4.5 Hz, 1H, H-6), 3.9 (s, 3H, $CO_2CH_3$), 3.8 (q, 2H, H-2), 2.1 (s, 3H, $CH_3$ imidazolidine) and 2.0 ppm (s, 3H, $CH_3$ imidazolidine).

## Claims

1. A process for the preparation of 3-chloro-cefem compounds, their salts and their derivatives of therapeutic or industrial application, of the general formula I

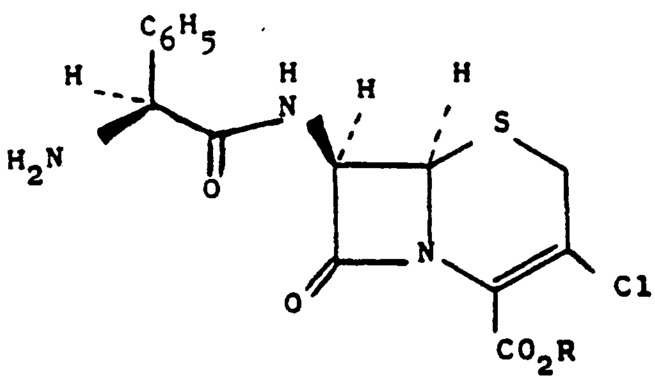

(I)

in which R represents hydrogen or a therapeutically acceptable metallic or organic cation, characterized in that it comprises the following stages:

(1) condensation of the compound of formula VII

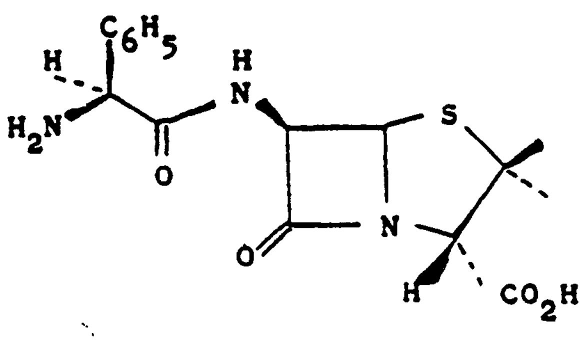

(VII)

with acetone in the presence of a basic medium, followed by a nitrosylation in an acid medium, to produce the formation of a compound of formula IX

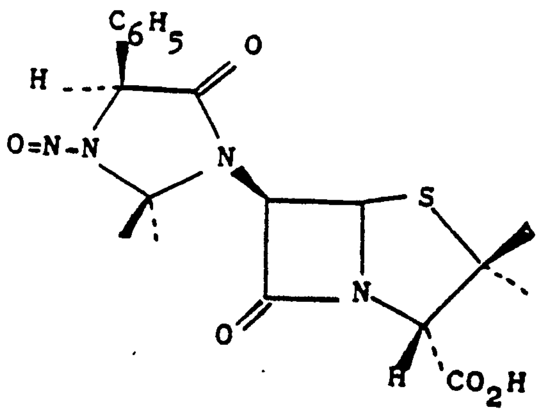

(IX)

(2) in either order subjecting the product of formula (IX) obtained in the previous stage tc esterification with a halogen derivative of the formula $R^2$-Hal, in which Hal represents halogen and $R^2$ is ar ester-forming alkyl or aryl group, and to oxidation, to produce the sulphoxide of the general formula XI

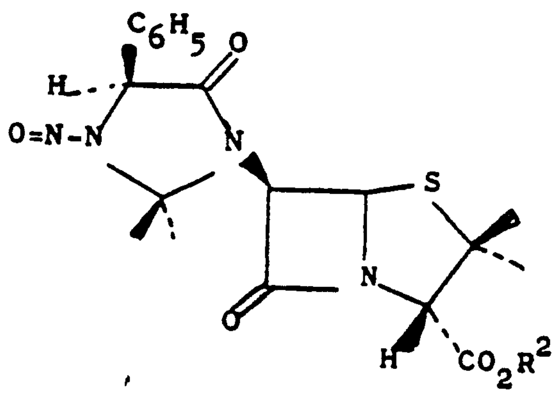

(X)

in which $R^2$ has the value indicated above;

(3) treatment of the sulphoxide of formula XI, obtained in the previous stage, first with a halogenatinç agent to obtain the intermediate sulphenyl chloride of formula XII

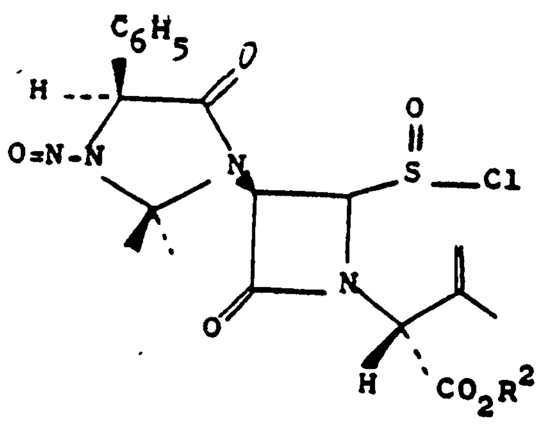

(XII)

in which $R^2$ is as defined above and Hal is a halogen atom, and subsequently with an acidic compound ii order finally to obtain the 3-exomethylcefam derivative of the general formula XIII

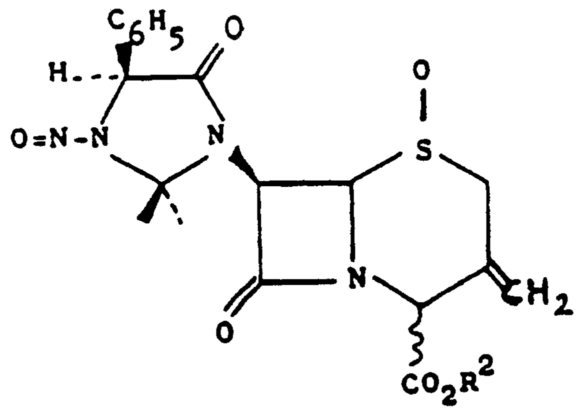

(XIII)

in which $R^2$ is as defined above;

(4) submitting the exomethylene bond of compound XIII, obtained in the previous stage, to oxidative cleavage, to obtain the 3-hydroxycefem derivative of the general formula XIV

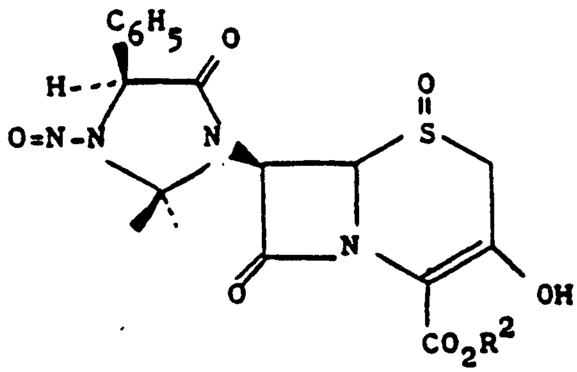

(XIV)

in which $R^2$ is as defined above;

(5) performing the following three reactions: (i) substitution of the hydroxyl group in position 3 by a chlorine atom, (ii) reduction of the sulphoxide group, and (iii) reduction of the N-nitroso group in the lateral chain, by which a compound of general formula XVI is obtained

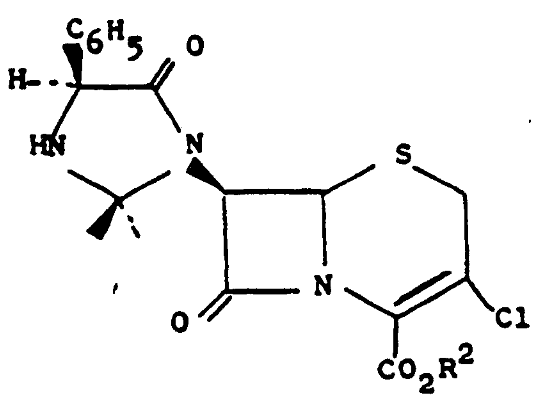

(XVI)

in which $R^2$ is as defined above, and

(6) de-protection of the side chain and the carboxyl group, finally separating the product of formula I.

2. A process according to Claim 1, characterized in that the basic medium used in Stage 1 is a tertiary amine.

3. A process according to Claim 1 or Claim 2, characterized in that the agent used in the nitrosylation in Stage 1 is an alkali metal salt of nitrous acid.

4. A process according to any of the preceding claims, characterized in that the agent used in the

esterification in Stage 2 is an alkyl or benzyl halide or derivatives thereof.

5. A process according to any of the preceding claims, characterized in that the esterification which is performed in Stage 2 takes place in the presence of a basic agent which is capable of capturing the hydrazides generated in the reaction medium.

6. A process according to Claim 5, characterized in that the basic agents used in the esterification in Stage 2 are bicarbonates of alkali metals.

7. A process according to any of the preceding claims, characterized in that the oxidation of the sulphide to sulphoxide in Stage 2 takes place using ozone as an oxidizing agent, under conditions in terms of solvents, concentration, temperature etc. which are customary in these reactions.

8. A process according to any of the preceding claims, characterized in that per-acids, such as peracetic acid or metachlorobenzoic acid, are used as oxidising agents in Stage 2.

9. A process according to any of the preceding claims, characterized in that the intermediate sulphenyl chloride is obtained in Stage 3 by treating the sulphoxide with halogenating agents of the type N-haloamides or N-haloimides.

10. A process according to any of the preceding claims, characterized in that the solvent used in Stage 3 belongs to the group of halogen derivatives, or to the hydrocarbon group.

11. A process according to any of the preceding claims, characterized in that the acid used to close the six-member ring in stage 3 is a compound of the group called Lewis acids.

12. A process according to any of the preceding claims, characterized in that the oxidative cleavage in Stage 4 is carried out by ozonolysis, i.e. treatment with ozone under the customary conditions of this reaction.

13. A process according to any of the preceding claims, characterized in that Stage 5 is carried out by using the reagent formed in the reaction of dimethylformamide with phosphorus trichloride.

14. A process according to any of the preceding claims, characterized in that the three reactions which constitute Stage 5 are carried out in a single step.

15. A process according to any of the preceding claims, characterized in that the de-protection of the acetonylidene group of the side chain is carried out by hydrolysis in an acid medium.

16. A process according to any of the preceding claims, characterized in that the de-protection of the ester group is carried out as a function of the nature of the esterifying group, either by hydrolysis or by reduction.

17. A process according to any of the preceding claims, characterized by the separation in an aqueous medium with an isoelectric pH of the compound of formula I in which R = H.